# EUROPÄISCHE PATENTSCHRIFT

(11) **EP 1 971 575 B1**
(45) Veröffentlichungstag und Bekanntmachung des Hinweises auf die Patenterteilung: **21.04.2010**
(21) Anmeldenummer: 07857476.1
(22) Anmeldetag: 12.12.2007
(51) Int. Cl.: C07D 209/58, C10L 1/00, C10M 171/00

(54) **ANTHRACHINONDERIVATE ALS MARKIERSTOFFE FÜR FLÜSSIGKEITEN**
ANTHRAQUINONE DERIVATIVES AS MARKERS FOR LIQUIDS
DÉRIVÉS D'ANTHRAQUINONE EN TANT QUE MARQUEURS POUR LIQUIDES

(30) Priorität: 20.12.2006 EP 06126725
(43) Veröffentlichungstag der Anmeldung: 24.09.2008
(73) Patentinhaber: BASF SE, 67056 Ludwigshafen (DE)
(72) Erfinder: SENS, Rüdiger, 67069 Ludwigshafen (DE); GESSNER, Thomas, 69120 Heidelberg (DE); EBERT, Sophia, 68165 Mannheim (DE); VAMVAKARIS, Christos, 68165 Mannheim (DE); AHLERS, Wolfgang, 67245 Lambsheim (DE)
(86) Internationale Anmeldenummer: PCT/EP2007/063813
(87) Internationale Veröffentlichungsnummer: WO 2008/074709

(56) Entgegenhaltungen:
- EP-A- 1 001 003
- EP-A- 1 479 749
- EP-A- 1 486 554

## Beschreibung

Die vorliegende Erfindung betrifft die Verwendung von Verbindungen der allgemeinen Formel (I) 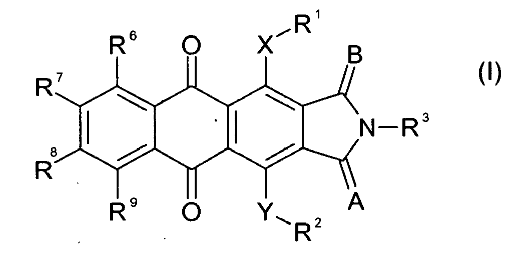 als Markierstoffe für Flüssigkeiten, wobei die Symbole folgende Bedeutung haben
- X, Y: unabhängig voneinander, gleich oder verschieden, O, NR⁴, Heterocyclen,
- A, B: unabhängig voneinander, gleich oder verschieden, O, NR⁵,
- M: Alkalimetalle,
- R¹, R²: unabhängig voneinander, gleich oder verschieden, H, C₁-C₂₀-Alkyl, C₂-C₂₀-Alkenyl, C₂-C₂₀-Alkinyl, C₃-C₁₅-Cycloalkyl, Aryl, Heterocyclen,
- R³: C₁-C₂₀-Alkyl, C₁-C₂₀-Alkylcarbonyl, C₂-C₂₀-Alkenyl, C₂-C₂₀-Alkenylcarbonyl , C₂-C₂₀-Alkinyl, C₂-C₂₀-Alkinylcarbonyl, C₃-C₁₅-Cycloalkyl, C₃-C₁₅-Cycloalkylcarbonyl, Aryl, Arylcarbonyl, Heterocyclen,
- R⁴: H, C₁-C₂₀-Alkyl, C₂-C₂₀-Alkenyl, C₂-C₂₀-Alkinyl,
- R⁵: C₃-C₁₅-Cycloalkyl, Aryl, Heterocyclen, H, C₁-C₂₀-Alkyl, C₂-C₂₀-Alkenyl, C₂-C₂₀-Alkinyl, C₃-C₁₅-Cycloalkyl, Aryl, Heterocyclen,
- R⁶, R⁷, R⁸, R⁹: unabhängig voneinander, gleich oder verschieden, H, C₁-C₂₀-Alkyl, C₁-C₂₀-Alkylcarbonyl, C₁-C₂₀-Alkoxy, C₁-C₂₀- Alkoxycarbonyl, C₂-C₂₀-Alkenyl, C₂-C₂₀-Alkenylcarbonyl , C₂- C₂₀-Alkinyl, C₂-C₂₀-Alkinylcarbonyl, C₃-C₁₅-Cycloalkyl, C₃-C₁₅-Cycloalkylcarbonyl, Aryl, Arylcarbonyl, Aryloxy, Aryloxycarbonyl, Heterocyclen, NR¹R², Halogen, CN, NO₂,

wobei die Substituenten R¹, R², R³, R⁴, R⁵, R⁶, R⁷, R⁸ oder R⁹ jeweils an beliebiger Position durch ein oder mehrere Heteroatome unterbrochen sein können, wobei die Anzahl dieser Heteroatome nicht mehr als 10, bevorzugt nicht mehr als 8, ganz besonders bevorzugt nicht mehr als 5 und insbesondere nicht mehr als 3 beträgt, und/oder jeweils an beliebiger Position, allerdings nicht mehr als fünfmal, bevorzugt nicht mehr als viermal und besonders bevorzugt nicht mehr als dreimal, durch NR¹R², CONR¹R², COOM, COOR¹, SO₃M, SO₃R¹, CN, NO₂, C₁-C₂₀-Alkyl, C₁-C₂₀-Alkoxy, Aryl, Aryloxy, Heterocyclen, Heteroatome oder Halogen substituiert sein können, wobei diese ebenfalls maximal zweimal, bevorzugt maximal einmal mit den genannten Gruppen substituiert sein können.

Die Erfindung betrifft weiterhin Flüssigkeiten, die eine Verbindung der allgemeinen Formel (I) als Markierstoff enthalten. Ferner betrifft die Erfindung Verfahren zur Detektion von Markierstoffen in Flüssigkeiten und zur Identifikation von Flüssigkeiten, welche mindestens eine Verbindung der allgemeinen Formel (I) enthalten. Weiterhin betrifft die Erfindung verschiedene neue Verbindungen der allgemeinen Formel (I).

Weitere Ausführungsformen der vorliegenden Erfindung sind den Ansprüchen, der Beschreibung und den Beispielen zu entnehmen. Es versteht sich, dass die vorstehend genannten und die nachstehend noch zu erläuternden Merkmale des erfindungsgemäßen Gegenstandes nicht nur in der jeweils konkret angegebenen Kombination, sondern auch in anderen Kombinationen verwendbar sind, ohne den Rahmen der Erfindung zu verlassen. Bevorzugt beziehungsweise ganz bevorzugt sind insbesondere auch diejenigen Ausführungsformen der vorliegenden Erfindung, in denen alle Merkmale des erfindungsgemäßen Gegenstandes die bevorzugten beziehungsweise ganz bevorzugten Bedeutungen haben.

Bestimmte Anthrachinonderivate und ihre Verwendung als Markierstoffe für Mineralölprodukte sind bekannt.

In der US 3,164,449 werden Anthrachinonderivate und ihre Verwendung als Markierstoffe für Mineralöle beschrieben. Die in dieser Schrift beschriebenen Anthrachinonderivate umfassen nicht Verbindungen der allgemeinen Formel (I).

EP 1 001 003 A1 beschreibt ein Verfahren zur unsichtbaren Markierung von Mineralölprodukten mit Hilfe von Farbstoffen. Diese Farbstoffe können unter anderem auch bestimmte 1,4-substituierte Anthrachinonderivate sein, die jedoch Verbindungen der allgemeinen Formel (I) nicht umfassen.

Die Schriften EP 1 323 811 A2, EP 1 422 284 A2, EP 1 426 434 A2, EP 1 479 749 A1 und EP 1 486 554 A1 offenbaren Methoden zur Markierung von Mineralölen durch Zugabe verschiedener Anthrachinonderivate. Es handelt sich hierbei beispielsweise um 1,4,5,8-tetrasubstituierte Anthrachinone bzw. Anthrachinondimere, mit Absorptionsmaxima im Bereich von 710 bis 850 nm, oder tetra- bis octasubstituierte Anthrachinone, mit Absorbtionsmaxima im Bereich von 690 bis 1000 nm. Mischungen verschiedener Anthrachinonderivate werden ebenfalls beschrieben. Die in diesen Schriften offenbarten Anthrachinonderivate umfassen nicht Verbindungen der allgemeinen Formel (I).

WO 2005/063942 A1 offenbart Kraft- und Schmierstoff-Konzentrate, welche mindestens ein Anthrachinonderivat als Markierungsstoff enthalten. Verbindungen der allgemeinen Formel (I) sind nicht als Markierstoffe beschrieben.

Bestimmte Verbindungen der allgemeinen Formel (I), insbesondere spezielle Anthrachinondicarbonsäureimide ("Anthrachinondicarboximide") und Verfahren zu deren Herstellung sind bekannt.

DE 939 044 und DE 945 112 beschreiben Verfahren zur Herstellung von am Imidstickstoff substituierten 1,4-Diamino-2,3-anthrachinondicarbonsäureimiden und deren Verwendung beim Färben von Polyethylenterephthalsäureester-Fasern. Eine Verwendung dieser Verbindungen als Markierstoffe für Flüssigkeiten ist nicht offenbart. DE 1 176 777 beschreibt die Herstellung von bestimmten Anthrachinondicarbonsäureimiden ausgehend von 1-Amino-4-nitroanthrachinon-2-carbonsäure. Eine Verwendung dieser Verbindungen als Markierstoffe für Flüssigkeiten ist nicht offenbart.

In der Praxis zeigt sich, dass viele der bekannten Markierstoffe, insbesondere in Mineralölen, mit den darin typischerweise vorhandenen Additiven, oder in Additiv-Konzentraten oftmals nicht die gewünschte Langzeitstabilität aufweisen. Durch die Einwirkung besagter Additive verändern sich beispielsweise die spektralen Eigenschaften (z.B. Extinktion) der Markierstoffe. Häufig ist daher eine genaue Detektion der Markierstoffe und eine zuverlässige Identifikation der Flüssigkeiten, insbesondere bei niedrigen Markierstoffkonzentrationen nach längeren Zeiträumen nur eingeschränkt möglich.

Aufgabe der Erfindung war es daher, weitere Anthrachinonderivate und verwandte Verbindungen zur Verfügung zu stellen, welche sich nicht nur durch gute Löslichkeit, sondern auch durch gute Langzeitbeständigkeit und Lagerbeständigkeit in den zu markierenden Flüssigkeiten, insbesondere Mineralölen oder Additiv-Konzentraten, auszeichnen.

Eine Detektion der Markierstoffe findet häufig mit Hilfe spektroskopischer Methoden durch Nachweis der Absorption oder der Fluoreszenz statt. Je höher die Fluoreszenzquantenausbeute der Markierstoffe ist, desto empfindlicher kann eine Detektion selbst bei niedrigen Konzentration des Markierungsstoffs erfolgen. Eine weitere Aufgabe der Erfindung war es daher Markierstoffe zu finden, die eine gegenüber den bekannten Markierstoffen, insbesondere den für die Markierung von Mineralölen verwendeten Anthrachinonen, erhöhte Quantenausbeute aufweisen.

Die Detektion der Markierstoffe kann bei unterschiedlichen Temperaturen erfolgen. Es ist daher nötig Markierstoffe zu finden, deren Detektion möglichst temperaturunabhängig oder mit reproduzierbarer bekannter Temperaturabhängigkeit erfolgen kann.

Es wurde gefunden, dass die oben beschriebenen Verbindungen der allgemeinen Formel (I), beispielsweise Anthrachinondicarbonsäureimide und verwandte Verbindungen, sowohl eine gute Löslichkeit, wie auch eine sehr gute Langzeitstabilität, insbesondere gegenüber üblichen Kraftstoffadditiven, aufweisen. Weiterhin zeichnen sich insbesondere Anthrachinondicarbonsäureimide, die unter die oben beschriebenen Verbindungen der allgemeinen Formel (I) fallen, durch eine erhöhte Fluoreszenzquantenausbeute gegenüber den im Stand der Technik als Mineralölmarker verwendeten Anthrachinonderivate aus. Die gefundene Temperaturabhängigkeit der Fluoreszenz ist bei den Verbindungen der allgemeinen Formel (I) häufig sehr gering.

Ausdrücke der Form Cₐ-C_{b} bezeichnen im Rahmen dieser Erfindung chemische Verbindungen oder Substituenten mit einer bestimmten Anzahl von Kohlenstoffatomen. Die Anzahl an Kohlenstoffatomen kann aus dem gesamten Bereich von a bis b, einschließlich a und b gewählt werden, a ist mindestens 1 und b immer größer als a. Eine weitere Spezifizierung der chemischen Verbindungen oder der Substituenten erfolgt durch Ausdrücke der Form Cₐ-C_{b}-V. V steht hierbei für eine chemische Verbindungsklasse oder Substituentenklasse, beispielsweise für Alkylverbindungen oder Alkylsubstituenten.

Halogen steht für Fluor, Chlor, Brom, oder Iod, vorzugsweise für Fluor, Chlor oder Brom, besonders bevorzugt für Fluor oder Chlor.

Alkalimetalle sind Li, Na oder K. Insbesondere können die Alkalimetalle (M) in der chemischen Gruppe -SO₃M oder -COOM als einwertige positiv geladene Ionen auftreten.

Im einzelnen haben die für die verschiedenen Substituenten R¹, R², R³, R⁴, R⁵, R⁶, R⁷, R⁸, R⁹, R¹⁰, R¹¹, X, Y, A und B angegebenen Sammelbegriffe folgende Bedeutung:
C₁-C₂₀-Alkyl: geradkettige oder verzweigte Kohlenwasserstoffreste mit bis zu 20 Kohlenstoffatomen, beispielsweise C₁-C₁₀-Alkyl oder C₁₁-C₂₀-Alkyl, bevorzugt C₁-C₁₀-Alkyl beispielsweise C₁-C₃-Alkyl, wie Methyl, Ethyl, Propyl, Isopropyl, oder C₄-C₆-Alkyl, n-Butyl, sec-Butyl, tert.-Butyl, 1,1-Dimethylethyl, Pentyl, 2-Methylbutyl, 1,1-Dimethylpropyl, 1,2-Dimethylpropyl, 2,2-Dimethylpropyl, 1-Ethylpropyl, Hexyl, 2-Methylpentyl, 3-Methyl-pentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 2-Ethylbutyl, 1,1,2-Trimethylpropyl, 1,2,2-Tri-methylpropyl, 1-Ethyl-1-methylpropyl, 1-Ethyl-2-methylpropyl, oder C₇-C₁₀-Alkyl, wie Heptyl, Octyl, 2-Ethyl-hexyl, 2,4,4-Trimethylpentyl, 1,1,3,3-Tetramethylbutyl, Nonyl oder Decyl sowie deren Isomere.
C₁-C₂₀-Alkylcarbonyl: eine geradkettige oder verzweigte Alkylgruppe mit 1 bis 20 Kohlenstoffatomen (wie vorstehend genannt), welche über eine Carbonylgruppe (-CO-) angebunden ist, bevorzugt C₁-C₁₀-Alkylcarbonyl wie beispielsweise Formyl, Acetyl, n-oder iso-Propionyl, n-, iso-, sec- oder tert.-Butanoyl, n-iso-, sec- oder tert.-Pentanoyl, n-oder iso-Nonanoyl, n-Dodecanoyl.
C₁-C₂₀-Alkoxy bedeutet eine geradkettige oder verzweigte Alkylgruppe mit 1 bis 20 Kohlenstoffatomen (wie vorstehend genannt), welche über ein Sauerstoffatom (-O-) angebunden sind, beispielsweise C₁-C₁₀-Alkoxy oder C₁₁-C₂₀-Alkoxy, bevorzugt C₁-C₁₀-Alkyloxy, insbesondere bevorzugt C₁-C₃-Alkoxy, wie beispielweise Methoxy, Ethoxy, Propoxy.
C₁-C₂₀-Alkoxycarbonyl: ist eine Alkoxygruppe mit 1 bis 20 Kohlenstoffatomen (wie vorstehend genannt), welche über eine Carbonylgruppe (-CO-) angebunden ist, bevorzugt C₁-C₁₀-Alkyloxycarbonyl.
C₂-C₂₀-Alkenyl: ungesättigte, geradkettige oder verzweigte Kohlenwasserstoffreste mit 2 bis 20 Kohlenstoffatomen und einer Doppelbindung in einer beliebigen Position, beispielsweise C₂-C₁₀-Alkenyl oder C₁₁-C₂₀-Alkenyl, bevorzugt C₂-C₁₀-Alkenyl wie C₂-C₄-Alkenyl, wie Ethenyl, 1-Propenyl, 2-Propenyl, 1-Methylethenyl, 1-Bute-nyl, 2-Butenyl, 3-Butenyl, 1-Methyl-1-propenyl, 2-Methyl-1-propenyl, 1-Methyl-2-pro-penyl, 2-Methyl-2-propenyl, oder C₅-C₆-Alkenyl, wie 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-bu-tenyl, 3-Methyl-2-butenyl, 1-Methyl-3-butenyl, 2-Methyl-3-butenyl, 3-Methyl-3-butenyl; 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl oder 1-Ethyl-2-methyl-2-propenyl, sowie C₇-C₁₀-Alkenyl, wie die Isomere von Heptenyl, Octenyl, Nonenyl oder Decenyl.
C₂-C₂₀-Alkenylcarbonyl: ungesättigte, geradkettige oder verzweigte Kohlenwasserstoffreste mit 2 bis 20 Kohlenstoffatomen und einer Doppelbindung in einer beliebigen Position (wie vorstehend genannt), welche über eine Carbonylgruppe (-CO-) angebunden sind, bevorzugt C₂-C₁₀-Alkylcarbonyl wie beispielsweise Ethenoyl, Propenoyl, Butenoyl, Pentenoyl, Nonenoyl sowie deren Isomere.
C₂-C₂₀-Alkinyl: geradkettige oder verzweigte Kohlenwasserstoffgruppen mit 2 bis 20 Kohlenstoffatomen und einer Dreifachbindung in einer beliebigen Position, beispielsweise C₂-C₁₀-Alkinyl oder C₁₁-C₂₀-Alkinyl, bevorzugt C₂-C₁₀-Alkinyl wie C₂-C₄-Alkinyl, wie Ethinyl, 1-Propinyl, 2-Propinyl, 1-Butinyl, 2-Butinyl, 3-Butinyl, 1-Me-thyl-2-propinyl, oder C₅-C₇-Alkinyl, wie 1-Pentinyl, 2-Pentinyl, 3-Pentinyl, 4-Pentinyl, 1-Methyl-2-butinyl, 1-Me-thyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl oder 1-Ethyl-1-methyl-2-propinyl sowie C₇-C₁₀-Alkinyl, wie die Isomere von Heptinyl, Octinyl, Noninyl, Decinyl.
C₂-C₂₀-Alkinylcarbonyl: ungesättigte, geradkettige oder verzweigte Kohlenwasserstoffreste mit 2 bis 20 Kohlenstoffatomen und einer Dreifachbindung in einer beliebigen Position (wie vorstehend genannt), welche über eine Carbonylgruppe (-CO-) angebunden sind, bevorzugt C₂-C₁₀-Alkinylcarbonyl wie beispielsweise Propinoyl, Butinoyl, Pentinoyl, Noninoyl, Decinoyl sowie deren Isomere.
C₃-C₁₅-Cycloalkyl: monocyclische, gesättigte Kohlenwasserstoffgruppen mit 3 bis zu 15 Kohlenstoffringgliedern, bevorzugt C₃-C₈-Cycloalkyl wie Cyclopropyl, Cyclobutyl, Cyclopentyl, Cyclohexyl, Cycloheptyl oder Cyclooctyl sowie ein gesättigtes oder ungesättigtes cyclisches System wie z. B. Norbornyl oder Norbenyl.
C₃-C₁₅-Cycloalkylcarbonyl: monocyclische, gesättigte Kohlenwasserstoffgruppen mit 3 bis 15 Kohlenstoffringgliedern (wie vorstehend genannt), welche über eine Carbonylgruppe (-CO-) angebunden sind, bevorzugt C₃-C₈-Cycloalkylcarbonyl.
Aryl: ein ein- bis dreikerniges aromatisches Ringsystem enthaltend 6 bis 14 Kohlenstoffringglieder, z. B. Phenyl, Naphthyl oder Anthracenyl, bevorzugt ein ein- bis zweikerniges, besonders bevorzugt ein einkerniges aromatisches Ringsystem.
Arylcarbonyl: bevorzugt ein ein- bis dreikerniges aromatisches Ringsystem (wie vorstehend genannt), welches über eine Carbonylgruppe (-CO-) angebunden ist, wie z. B. Benzoyl, bevorzugt ein ein- bis zweikerniges, besonders bevorzugt ein einkerniges aromatisches Ringsystem.
Aryloxy: ist ein ein- bis dreikerniges aromatisches Ringsystem (wie vorstehend genannt), welches über ein Sauerstoffatom (-O-) angebunden ist, bevorzugt ein ein- bis zweikerniges, besonders bevorzugt ein einkerniges aromatisches Ringsystem.
Aryloxycarbonyl: ist eine ein- bis dreikemige Aryloxygruppe (wie vorstehend genannt), welche über eine Carbonylgruppe (-CO-) angebunden ist, bevorzugt ein ein- bis zweikerniges, besonders bevorzugt ein einkerniges Aryloxycarbonyl.
Heterocyclen: fünf- bis zwölfgliedrige, bevorzugt fünf- bis neungliedrige, besonders bevorzugt fünf- bis sechsgliedrige, Sauerstoff-, Stickstoff- und/oder Schwefelatome, gegebenenfalls mehrere Ringe aufweisende Ringsysteme wie Furyl, Thiophenyl, Pyrryl, Pyridyl, Indolyl, Benzoxazolyl, Dioxolyl, Dioxyl, Benzimidazolyl, Benzthiazolyl, Dimethylpyridyl, Methylchinolyl, Dimethylpyrryl, Methoxyfuryl, Dimethoxypyridyl, Difluorpyridyl, Methylthiophenyl, Isopropylthiophenyl oder tert.-Butylthiophenyl. Die Heterocyclen können in beliebiger Weise chemisch an die Verbindungen der allgemeinen Formel (I) angebunden sein, beispielsweise über eine Bindung zu einem Kohlenstoffatom des Heterocyclus oder eine Bindung zu einem der Heteroatome. Weiterhin, insbesondere, fünf- oder sechsgliedrige gesättigte stickstoffhaltige Ringsysteme, die über ein Ringstickstoffatom angebunden sind und die noch ein oder zwei weitere Stickstoffatome oder ein weiteres Sauerstoff- oder Schwefelatom enthalten können.
COOR¹: steht für Carbonsäuren (R¹ = H) oder Carbonsäureester (mit beispielsweise R¹= C₁-C₂₀-Alkyl oder Aryl).
   COOM: steht für Salze von Carbonsäuren (beispielsweise einwertige Alkalimetallsalze).
SO₃R¹: steht für Sulfonsäuren (R¹ = H) oder Sulfonsäureester (mit beispielsweise R¹= C₁-C₂₀-Alkyl oder Aryl).
   SO₃M: steht für Salze von Sulfonsäuren (beispielsweise einwertige Alkalimetallsalze).
CONR¹R²: steht für gegebenenfalls substituierte Carbonsäureamide. Beispielsweise sind in diesem Fall R¹ und R² gleich oder verschieden C₁-C₂₀-Alkyl oder Aryl.
Heteroatome sind Phosphor, Sauerstoff, Stickstoff oder Schwefel bevorzugt Sauerstoff, Stickstoff oder Schwefel.

Bevorzugt haben die Symbole in Formel (I) folgende Bedeutung:
- X, Y: unabhängig voneinander, gleich oder verschieden, O, NR⁴,
- A, B: unabhängig voneinander, gleich oder verschieden, NH, O,
- R¹, R²: gleich oder verschieden H, C₁-C₂₀-Alkyl, Aryl,
- R³: C₁-C₂₀-Alkyl, C₃-C₁₅-Cycloalkyl, Aryl, Heterocyclen,
- R⁴,: H, C₁-C₂₀-Alkyl, Aryl, Heterocyclen,
- R⁶, R⁷, R⁸, R⁹: unabhängig voneinander, gleich oder verschieden, H, C₁-C₂₀-Alkyl, C₁-C₂₀-Alkoxy, C₃-C₁₅-Cycloalkyl, Aryl, Aryloxy, NR¹R², Halogen, CN, NO₂.

Ganz bevorzugt haben die Symbole in Formel (I) folgende Bedeutung:
- X, Y: beide gleich NR⁴,
- A, B: unabhängig voneinander, gleich oder verschieden, NH, O,
- R¹, R²: H,
- R³: C₁-C₂₀-Alkyl, C₃-C₁₅-Cycloalkyl, Aryl,
- R⁴,: H,
- R⁶, R⁷, R⁸, R⁹: unabhängig voneinander, gleich oder verschieden, H, C₁-C₂₀-Alkyl, C₁-C₂o-Alkoxy, Aryl, Aryloxy, NR¹R², F, Cl, Br, CN, NO₂.

Ebenfalls bevorzugt haben die Symbole in Formel (I) folgende Bedeutung:
- X, Y: beide gleich NR⁴,
- A, B: O,
- R¹, R²: H,
- R³: C₁-C₂₀-Alkyl, C₃-C₁₅-Cycloalkyl, Aryl,
- R⁴,: H,
- R⁶, R⁷, R⁸, R⁹: unabhängig voneinander, gleich oder verschieden, H, C₁-C₂₀-Alkyl, C₁-C₂₀-Alkoxy, Aryl, Aryloxy, NR¹R², F, Cl, Br, CN, NO₂.

Bevorzugt bzw. ganz bevorzugt ist die Verwendung von Verbindungen der allgemeinen Formel (I), in denen alle Symbole die bevorzugten bzw. ganz bevorzugten Bedeutungen haben.

Als Markierstoffe, können im Rahmen des erfindungsgemäßen Verfahrens sowohl einzelne Verbindungen der allgemeinen Formel (I) oder Gemische von Verbindungen der allgemeinen Formel (I) eingesetzt werden.

Die Verbindungen der allgemeinen Formel (I) können nach dem Fachmann geläufigen Methoden hergestellt werden, wie sie beispielsweise in M. C. Marschalk, Bull. Soc. Chim. 1937, 184 - 193, DE 1 769 470, DE 1 176 777, DE 939 044 und DE 945 112 beschrieben sind.

Die Verbindungen der allgemeinen Formel (I) sind teilweise bekannt und teilweise neu.

Gegenstand der Erfindung sind daher unter anderem auch Verbindungen der allgemeinen Formel (I) bei denen die Symbole in Formel (I) folgende Bedeutung haben:
- X, Y: NH,
- A, B: O,
- R¹, R²: H,
- R³: C₁₃-C₂₀-Alkyl,
- R⁶, R⁷, R⁸, R⁹: H.

Insbesondere bevorzugt ist Gegenstand der Erfindung die Verbindung mit R³=C₁₃-Alkyl: 1,4-Diamino-N-tridecyl-2,3-anthrachinondicarboximid. Der Tridecyl-Substituent in dieser Verbindung kann natürlich auch ein Isomerengemisch verschiedener Tridecyle darstellen.

Weiterhin bevorzugt sind Gegenstand der Erfindung die Verbindungen 1,4-Diamino-N-(2,6-düsopropylphenyl)-2,3-anthrachinondicarboximid oder 1,4-Diamino-N-(4-dodecylphenyl)-2,3-anthrachinondicarboximid, sowie Mischungen dieser Verbindungen. Der Dodecyl-Substituent in der Verbindung 1,4-Diamino-N-(4-dodecylphenyl)-2,3-anthrachinondicarboximid kann natürlich auch ein Isomerengemisch verschiedener Dodecyle darstellen.

Geeignete Flüssigkeiten, die mittels der Verbindungen der allgemeinen Formel (I) entsprechend dem erfindungsgemäßen Verfahren markiert werden können, sind insbesondere Wasser oder organische Flüssigkeiten, beispielsweise Alkohole, wie Methanol, Ethanol, Propanol, Isopropanol, Butanol, Isobutanol, sec-Butanol, Pentanol, Isopentanol, Neopentanol oder Hexanol, Glykole, wie 1,2-Ethylenglykol, 1,2- oder 1,3-Propylenglykol, 1,2-, 2,3- oder 1,4-Butylenglykol, Di- oder Triethylenglykol oder Di- oder Tripropylenglykol, Ether, wie Methyl-tert.-butylether, 1,2-Ethylenglykolmono- oder - dimethylether, 1,2-Ethylenglykolmono- oder -diethylether, 3-Methoxypropanol, 3-Isopropoxypropanol, Tetrahydrofuran oder Dioxan, Ketone, wie Aceton, Methylethylketon oder Diacetonalkohol, Ester, wie Essigsäuremethylester, Essigsäureethylester, Essigsäurepropylester oder Essigsäurebutylester, aliphatische oder aromatische Kohlenwasserstoffe wie Pentan, Hexan, Heptan, Octan, Isooctan, Petrolether, Toluol, Xylol, Ethylbenzol, Tetralin, Dekalin, Dimethylnaphthalin, Testbenzin, Bremsflüssigkeiten oder Öle, wie Mineralöle, die erfindungsgemäß Benzin, Kerosin, Dieselöl und Heizöl umfassen, natürliche Öle, wie Olivenöl, Sojaöl oder Sonnenblumenöl, oder natürliche oder synthetische Motoren-, Hydraulik- oder Getriebeöle, z.B. Fahrzeugmotorenöl oder Nähmaschinenöl.

Besonders vorteilhaft verwendet man die Verbindungen der allgemeinen Formel (I) entsprechend dem erfindungsgemäßen Verfahren zum Markieren von Ölen, insbesondere Mineralölen.

Gegenstand der Erfindung sind des Weiteren Flüssigkeiten, bevorzugt Öle, insbesondere Mineralöle, welche mindestens eine Verbindung der allgemeinen Formel (I) als Markierungsstoff enthalten.

Die als Markierstoffe zu verwendenden Verbindungen der allgemeinen Formel (I) werden den Flüssigkeiten in solchen Mengen zugegeben, dass eine zuverlässige Detektion gewährleistet ist. Üblicherweise beträgt der (gewichtsbezogene) Gesamtgehalt an Markierstoffen in der markierten Flüssigkeit etwa 0,1 bis 5000 ppb, bevorzugt 1 bis 2000 ppb und besonders bevorzugt 1 bis 1000 ppb.

Zur Markierung der Flüssigkeiten werden die Verbindungen im Allgemeinen in Form von Lösungen (Stammlösungen) zugegeben. Insbesondere bei Mineralölen eignen sich als Lösungsmittel zur Bereitung dieser Stammlösungen vorzugsweise aromatische Kohlenwasserstoffe, wie Toluol, Xylol oder höhersiedende Aromatengemische.

Um eine zu hohe Viskosität solcher erfindungsgemäßer Stammlösungen (und damit schlechte Dosier- und Handhabbarkeit) zu vermeiden, wählt man im allgemeinen eine Gesamtkonzentration der Markierstoffe von 0,5 bis 50 Gew.-%, bevorzugt 0,5 bis 40 Gew.-%, besonders bevorzugt, 0,5 bis 30 Gew.-% bezogen auf das Gesamtgewicht dieser Stammlösungen.

Die Verbindungen der allgemeinen Formel (I) können gegebenenfalls auch in Mischung mit anderen Markierstoffen/Farbstoffen verwendet werden. Die Gesamtmenge der Markierstoffe in den Flüssigkeiten liegt dann üblicherweise im zuvor beschriebenen Bereich.

Gegenstand der Erfindung ist auch ein Verfahren zur Markierung von Flüssigkeiten, vorzugsweise Ölen, insbesondere Mineralölen, wobei der Flüssigkeit eine Verbindung der allgemeinen Formel (I) zugesetzt wird.

Gegenstand der Erfindung ist auch ein Verfahren zur Detektion von Markierstoffen in Flüssigkeiten, welche mindestens eine Verbindung der allgemeinen Formel (I) enthalten.

Die Detektion der Verbindungen der allgemeinen Formel (I) in den Flüssigkeiten erfolgt nach dem Fachmann bekannten gängigen Methoden. Da die Verbindungen der allgemeinen Formel (I) in der Regel ein hohes Absorptionsvermögen aufweisen und/oder hohe Fluoreszenzquantenausbeute zeigen, bietet sich im gegebenen Fall z.B. ein spektroskopischer Nachweis an. In diesem Zusammenhang wird explizit Bezug genommen auf die Offenbarung der Schriften WO 94/02570 (S. 14, Z. 10-46 und Abbildung 1), WO 99/55805 (S.22, Z. 7 - S. 34, Z. 46) und WO 99/56125 (S.22, Z. 22 - S. 46, Z. 15).

Die Verbindungen der allgemeinen Formel (I) weisen in der Regel ihr Absorptionsmaximum im Bereich von 500 bis 900 nm auf und/oder fluoreszieren im Bereich von 500 bis 1000 nm und können so mit geeigneten Instrumenten leicht detektiert werden.

Der Nachweis kann auf an sich bekannte Weise, zum Beispiel durch Messung des Absorptionsspektrums der zu untersuchenden Flüssigkeiten, erfolgen.

Man kann aber auch vorteilhaft die Fluoreszenz der in den Flüssigkeiten enthaltenen Verbindungen der allgemeinen Formel (I) anregen, vorteilhaft mit einem Halbleiterlaser oder einer Halbleiterdiode. Besonders günstig ist es, dabei einen Halbleiterlaser oder eine Halbleiterdiode mit einer Wellenlänge im Spektralbereich von λₘₐₓ -100 nm bis λₘₐₓ +20 nm anzuwenden. λₘₐₓ bedeutet dabei die Wellenlänge des längstwelligen Absorptionsmaximums des Markierstoffs. Die Wellenlänge der maximalen Emission liegt dabei in der Regel im Bereich von 500 bis 900 nm.

Das so erzeugte Fluoreszenzlicht wird vorteilhaft mit einem Halbleiterdetektor, insbesondere mit einer Silicium-Photodiode oder einer Germanium-Photodiode, detektiert.

Besonders vorteilhaft gelingt der Nachweis, wenn sich vor dem Detektor noch ein Interferenzfilter und/oder ein Kantenfilter (mit einer kurzwelligen Transmissionskante im Bereich von λₘₐₓ bis λₘₐₓ +80 nm) und/oder ein Polarisator befindet.

Mittels der oben genannten Verbindungen gelingt es sehr einfach, markierte Flüssigkeiten nachzuweisen, selbst wenn die Verbindungen der allgemeinen Formel (I) nur in einer Konzentration von ungefähr 1 ppm (Nachweis durch Absorption) oder ungefähr 100 ppb (Nachweis durch Fluoreszenz) vorliegen.

Ein bevorzugtes Verfahren zur Detektion von Markierstoffen in Flüssigkeiten, welche mindestens eine Verbindung der allgemeinen Formel (I) in einer Menge enthalten, die ausreichend ist, bei Bestrahlung mit Strahlung einer geeigneten Wellenlänge eine nachweisbare Fluoreszenz anzuregen, wird ausgeführt indem:
a) die Flüssigkeit mit elektromagnetischer Strahlung einer Wellenlänge von 500 bis 900 nm bestrahlt wird und
b) die angeregte Fluoreszenzstrahlung mit einer Vorrichtung zum Nachweis von Strahlung im Bereich von 500 bis 1000 nm detektiert wird.

Ein weiteres bevorzugtes Verfahren zur Detektion von Markierstoffen in Flüssigkeiten, welche mindestens eine Verbindung der allgemeinen Formel (I) in einer Menge enthalten, die ausreichend ist, bei Bestrahlung mit Strahlung einer geeigneten Wellenlänge eine nachweisbare Absorption zu zeigen, wird ausgeführt indem:
a) die Flüssigkeit mit elektromagnetischer Strahlung einer Wellenlänge von 500 bis 900 nm bestrahlt wird und
b) die Absorption der Strahlung a) mit einer Vorrichtung zum Nachweis von Strahlung im Bereich von 500 bis 900 nm detektiert wird.

Gegenstand der Erfindung ist auch ein Verfahren zur Identifizierung von Flüssigkeiten, vorzugsweise Ölen, insbesondere Mineralölen, welche eine Verbindung der allgemeinen Formel (I) in einer Menge enthalten, die ausreichend ist, bei Bestrahlung mit einer geeigneten Wellenlänge eine nachweisbare Fluoreszenz anzuregen, wobei
a) die Flüssigkeit mit elektromagnetischer Strahlung einer Wellenlänge von 500 bis 900 nm bestrahlt wird und
b) die Absorption der elektromagnetischen Strahlung a) mit einer Vorrichtung zum Nachweis von Strahlung detektiert wird und
c) die angeregte Fluoreszenzstrahlung mit einer Vorrichtung zum Nachweis von Strahlung im Bereich von 500 bis 900 nm detektiert wird und
d) die Flüssigkeit mit Hilfe der Absorption b) und/oder Fluoreszenz c) identifiziert wird und
e) die Konzentration der Verbindung der allgemeinen Formel (I) in der Flüssigkeit mit Hilfe der Fluoreszenzstrahlung c) bestimmt wird.

In einer bevorzugten Ausführungsform des erfindungsgemäßen Verfahrens zur Identifizierung werden die Messdaten aus den Schritten b) und e) des Verfahrens verknüpft um die Identifizierung durchzuführen. Die Identifizierung kann als weiteren Schritt den Abgleich mit bekannten spektroskopischen Daten enthalten. Beispielsweise handelt es sich bei den bekannten spektroskopischen Daten um elektronisch gespeicherte Spektren, die beispielweise in Datenbaken abgelegt sein können.

Falls Gemische von Verbindungen der allgemeinen Formel (I) als Markierstoff eingestetzt werden, so unterscheiden sich bevorzugt die Wellenlängenlagen der Absorptionsmaxima λₘₐₓ um einen spektroskopisch messbaren Betrag. Bevorzugt unterscheiden sich die Lagen der Absorptionsmaxima jeweils um den Betrag von mindestens 40 nm. λₘₐₓ bedeutet dabei die Wellenlänge des längstwelligen Absorptionsmaximums des Markierstoffs.

Im allgemeinen können die Absorptionsbanden der Verbindungen der allgemeinen Formel (I) entweder überlappen oder getrennt voneinander vorliegen. Durch gezielte Detektion und den kombinierten Nachweis mehrerer spektroskopischer Eigenschaften, beispielsweise in Absorption oder Fluoreszenz, lassen sich bei der erfindungsgemäßen Verwendung von Mischungen der Verbindungen der allgemeinen Formel (I) sogenannte "Fingerprint Systeme" aufbauen.

Bevorzugt lassen sich auch unterschiedliche Mengenverhältnisse in einem Gemisch der Verbindungen der allgemeinen Formel (I) dazu nutzen unterschiedliche Markierstoffe zu erhalten. Bevorzugt lassen sich mit zwei Verbindungen V¹ und V² der allgemeinen Formel (I), die in n unterschiedlichen Mengenverhältnissen vorliegen n Markierstoffe zur erfindungsgemäßen Verwendung erhalten (n ist eine ganze Zahl größer 1). Beispielsweise lassen sich im Falle von n=2, mit den Mengenverhältnissen V¹ : V² = 1:2 und V¹:V² = 2:1, zwei Markierstoffe erhalten. Die jeweiligen Mengenverhältnisse kann der Fachmann an Hand von einfachen Routineversuchen für die jeweilige Anwendung feststellen.

In einer bevorzugten Ausführungsform des erfindungsgemäßen Verfahrens werden neben Verbindungen der allgemeinen Formel (I) weitere (mindestens ein), von den Verbindungen der allgemeinen Formel (I) verschiedene, Markierungssubstanzen (MA) als Markierstoff eingesetzt. Bevorzugt werden in einem solchen Gemisch solche Markierungsstoffe (MA) eingesetzt, deren Absorptionsmaximum λₘₐₓ bei einer Wellenlänge liegt, die sich um mindestens 40 nm von der Lage der Absorptionsmaxima der im Gemisch auftretenden Verbindungen der allgemeinen Formel (I) unterscheidet. Bevorzugt werden in einem solchen Gemisch Markierungsstoffe (MA) eingesetzt, deren λₘₐₓ bei einer Wellenlänge liegt, die größer (längerwellig) ist als die der Verbindungen der allgemeinen Formel (I). Es lassen sich jedoch auch in einem solchen Gemisch Markierungsstoffe (MA) eingesetzen, deren λₘₐₓ bei einer Wellenlänge liegt, die kleiner (kürzerwellig) ist als die der Verbindungen der allgemeinen Formel (I).

Im allgemeinen können die Absorptionsbanden der Markierungsstoffe (MA) und der Verbindungen der allgemeinen Formel (I) entweder überlappen oder getrennt voneinander vorliegen. Durch gezielte Detektion und den kombinierten Nachweis mehrerer spektroskopischer Eigenschaften, beispielsweise in Absorption oder Fluoreszenz, lassen sich bei der erfindungsgemäßen Verwendung von Mischungen der Markierungsstoffe (MA) und der Verbindungen der allgemeinen Formel (I) sogenannte "Fingerprint Systeme" aufbauen.

Bevorzugt lassen sich auch unterschiedliche Mengenverhältnisse in einem Gemisch der Markierungsstoffe (MA) und der Verbindungen der allgemeinen Formel (I) dazu nutzen unterschiedliche Markierstoffe zu erhalten. Bevorzugt lassen sich mit zwei Verbindungen MA¹ der Markierungsstoffe (MA) und V² der allgemeinen Formel (I), die in n unterschiedlichen Mengenverhältnissen vorliegen n Markierstoffe zur erfindungsgemäßen Verwendung erhalten (n ist eine ganze Zahl größer 1). Beispielsweise lassen sich im Falle von n=2, mit den Mengenverhältnissen MA¹ : V² = 1:2 und MA¹:V² = 2:1, zwei Markierstoffe erhalten.

Das Mengenverhältnis von Markierungsstoffen (MA) zu Verbindungen der allgemeinen Formel (I) ist im allgemeinen abhängig von der jeweiligen Anwendung und der Nachweisempfindlichkeit des Markierungsstoffes (MA). Die jeweiligen Mengenverhältnisse kann der Fachmann an Hand von einfachen Routineversuchen für die jeweilige Anwendung feststellen.

Als mögliche Markierungsstoffe (MA) sind von den Verbindungen der allgemeinen Formel (I) verschiedene Anthrachinone, Phthalocyanine (metallfrei und metallhaltig) oder Naphthalocyanine zu nennen. Bevorzugt werden von den Verbindungen der allgemeinen Formel (I) verschiedene Anthrachinone oder Phthalocyanine und besonders bevorzugt Phthalocyanine eingesetzt.

Die Verbindungen der allgemeinen Formel (I) können auch als Komponente in Additiv-Konzentraten (im Folgenden, dem einschlägigen Sprachgebrauch folgend auch "Packages" genannt) zur Anwendung kommen, welche neben einem Trägeröl und einem Gemisch verschiedener Kraftstoffadditive in der Regel auch Farbstoffe sowie, zur unsichtbaren fiskalischen oder herstellerspezifischen Markierung, zusätzlich noch Markierstoffe enthalten. Diese Packages ermöglichen es, dass sich verschiedene Mineralölvertriebsfirmen aus einem "Pool" von unadditiviertem Mineralöl versorgen können und erst mit Hilfe ihrer individuellen Packages dem Mineralöl, z.B. während der Abfüllung in entsprechende Transportbehälter, die firmenspezifische Additivierung, Farbigkeit sowie Markierung verleihen.

Solche erfindungsgemäßen Packages enthalten dann als Komponenten insbesondere:
a) mindestens eine Verbindung der allgemeinen Formel (I),
b) mindestens ein Trägeröl,
c) mindestens ein Additiv ausgewählt aus der Gruppe bestehend aus
   i. Detergenzien,
   ii. Dispergatoren und
   iii. Ventilsitzverschleiß-hemmenden Additiven,
d) sowie gegebenenfalls weitere Additive und Hilfsmittel.

Als Trägeröle werden üblicherweise viskose, hochsiedende und insbesondere thermostabile Flüssigkeiten verwendet. Sie überziehen die heißen Metalloberflächen, z. B. die Einlassventile, mit einem dünnen Flüssigkeitsfilm und verhindern oder verzögern dadurch die Bildung und Ablagerung von Zersetzungsprodukten an den Metalloberflächen.

Als Komponente b) der Kraft- und Schmierstoffadditiv-Konzentrate in Frage kommende Trägeröle sind beispielsweise mineralische Trägeröle (Grundöle), insbesondere solche der Viskositätsklasse "Solvent Neutral (SN) 500 bis 2000", synthetische Trägeröle auf Basis von Olefinpolymerisaten mit M_{N} = 400 bis 1800, vor allem auf Polybuten- oder Polyisobuten-Basis (hydriert oder nicht hydriert), von Polyalphaolefinen oder Polyinternalolefinen sowie synthetische Trägeröle auf Basis alkoxylierter langkettiger Alkohole oder Phenole. Als Trägeröle zu verwendende Addukte von Ethylenoxid, Propylenoxid und/oder Butylenoxid an Polybutyl- oder Polyisobutenalkohole sind etwa in der Schrift EP 277 345 A1, weitere zu verwendende Polyalkenalkohol-Polyalkoxylate in der Schrift WO 00/50543 A1 beschrieben. Als weitere zu verwendende Trägeröle sind auch Polyalkenalkohol-Polyetheramine zu nennen, wie sie in der Schrift WO 00/61708 aufgeführt sind.

Selbstverständlich können auch Mischungen verschiedener Trägeröle zum Einsatz kommen, sofern sie untereinander und mit den übrigen Komponenten der Packages verträglich sind.

Vergaser und Einlasssysteme von Verbrennungsmotoren, aber auch Einspritzsysteme für die Kraftstoffdosierung, werden in zunehmendem Maße durch Verunreinigungen belastet, die beispielsweise durch Staubteilchen aus der Luft und unverbrannte Kohlenwasserstoffreste aus dem Brennraum verursacht werden.

Zur Reduzierung oder Vermeidung dieser Verunreinigungen werden dem Kraftstoff Additive ("Detergenzien") zur Reinhaltung von Ventilen und Vergasern beziehungsweise Einspritzsystemen beigegeben. Derartige Detergenzien gelangen im Allgemeinen in Kombination mit einem oder mehreren Trägerölen zur Anwendung. Die Trägeröle üben eine zusätzliche "Waschfunktion" aus, unterstützen und fördern oft die Detergenzien in ihrer reinigenden und reinerhaltenden Wirkung und können so zur Reduzierung der benötigten Menge an Detergenzien beitragen.

Erwähnt sei hier weiter, dass viele der üblicherweise als Trägeröle verwendeten Substanzen zusätzliche Wirkung als Detergenzien und/oder Dispergatoren entfalten, weshalb in einem solchen Fall der Anteil an letzteren reduziert werden kann. Solche Trägeröle mit Detergens-/Dispergatorwirkung sind etwa in der letztgenannten WO-Schrift dargelegt.

Auch lässt sich die Wirkungsweise von Detergenzien, Dispergatoren und Ventilsitzverschleiß-hemmenden Additiven oftmals nicht klar voneinander abgrenzen, weshalb diese Verbindungen summarisch unter Komponente c) aufgeführt sind. Übliche Detergenzien, welche in den Packages Anwendung finden, sind beispielsweise in den Schriften WO 00/50543 A1 und WO 00/61708 A1 aufgeführt und umfassen:
Polyisobutenamine, welche gemäß der EP-A 244 616 durch Hydroformylierung von hochreaktivem Polyisobuten und anschließende reduktive Aminierung mit Ammoniak, Monoaminen oder Polyaminen wie Dimethylenaminopropylamin, Ethylendiamin, Diethylentriamin, Triethylentetramin oder Tetraethylenpentamin erhältlich sind,
Poly(iso)butenamine, welche durch Chlorierung von Polybutenen oder Polyisobutenen mit Doppelbindungen überwiegend in der β- und γ-Position und anschließende Aminierung mit Ammoniak, Monoaminen oder den oben genannten Polyaminen erhältlich sind,
Poly(iso)butenamine, welche durch Oxidation von Doppelbindungen in Poly(iso)-butenen mit Luft oder Ozon zu Carbonyl- oder Carboxylverbindungen und anschließende Aminierung unter reduzierenden (hydrierenden) Bedingungen erhältlich sind,
Polyisobutenamine, welche gemäß der DE-A 196 20 262 aus Polyisobutenepoxiden durch Umsetzung mit Aminen und nachfolgende Dehydratisierung und Reduktion der Aminoalkohole erhältlich sind,
gegebenenfalls Hydroxylgruppen enthaltende Polyisobutenamine, welche gemäß der WO-A 97/03946 durch Umsetzung von Polyisobutenen mit einem mittleren Polymerisationsgrad P = 5 bis 100 mit Stickoxiden oder Gemischen aus Stickoxiden und Sauerstoff und anschließende Hydrierung dieser Umsetzungsprodukte erhältlich sind,
Hydroxylgruppen enthaltende Polyisobutenamine, welche gemäß der EP-A 476 485 durch Umsetzung von Polyisobutenepoxiden mit Ammoniak, Monoaminen oder den oben genannten Polyaminen erhältlich sind,
Polyetheramine, welche durch Umsetzung von C₂-C₃₀-Alkanolen, C₆-C₃₀-Alkandiolen, Mono- oder Di-C₂-C₃₀-Alkylaminen, C₁-C₃₀-Alkylcyclohexanolen oder C₁-C₃₀-Alkylphenolen mit 1 bis 30 mol Ethylenoxid und/oder Propylenoxid und/oder Butylenoxid pro Hydroxyl- beziehungsweise Aminogruppe und anschließende reduktive Aminierung mit Ammoniak, Monoaminen oder den oben genannten Polyaminen erhältlich sind, sowie
"Polyisobuten-Mannichbasen", welche gemäß EP-A 831 141 durch Umsetzung von polyisobutensubstituierten Phenolen mit Aldehyden und Monoaminen oder den oben genannten Polyaminen erhältlich sind.

Weitere zu verwendende Detergenzien und/oder Ventilsitzverschleiß-hemmende Additive sind beispielsweise in der Schrift WO 00/47698 A1 aufgeführt und umfassen Verbindungen, welche mindestens einen hydrophoben Kohlenwasserstoffrest mit einem zahlengemittelten Molekulargewicht (M_{N}) von 85 bis 20 000 und mindestens eine polare Gruppierung aufweisen, und welche ausgewählt sind aus:
(i) Mono- oder Polyaminogruppen mit bis zu 6 Stickstoffatomen, wobei mindestens ein Stickstoffatom basische Eigenschaften hat,
(ii) Nitrogruppen, gegebenenfalls in Kombination mit Hydroxylgruppen,
(iii) Hydroxylgruppen in Kombination mit Mono- oder Polyaminogruppen, wobei mindestens ein Stickstoffatom basische Eigenschaften hat,
(iv) Carboxylgruppen oder deren Alkalimetall- oder Erdalkalimetallsalzen,
(v) Sulfonsäuregruppen oder deren Alkalimetall- oder Erdalkalimetallsalzen,
(vi) Polyoxy-C2-C4-alkylengruppierungen, die durch Hydroxylgruppen, Mono- oder Polyaminogruppen, wobei mindestens ein Stickstoffatom basische Eigenschaften hat, oder durch Carbamatgruppen terminiert sind,
(vii) Carbonsäureestergruppen,
(viii) aus Bernsteinsäureanhydrid abgeleitete Gruppierungen mit Hydroxy- und/oder Amino- und/oder Amido- und/oder Imidogruppen und
(ix) durch Mannich-Umsetzung von phenolischen Hydroxylgruppen mit Aldehyden und Mono- oder Polyaminen erzeugte Gruppierungen.

Mono- oder Polyaminogruppen (i) enthaltende Additive sind vorzugsweise Polyalkenmono- oder Polyalkenpolyamine auf Basis von Polypropen oder von hochreaktivem (d.h. mit überwiegend endständigen Doppelbindungen - meist in der β- und γ-Position). oder konventionellem (d.h. mit überwiegend mittenständigen Doppelbindungen) Polybuten oder Polyisobuten mit M_{N} = 300 bis 5000. Derartige Additive auf Basis von hochreaktivem Polyisobuten, welche aus dem Polyisobuten, welches bis zu 20 Gew.-% n-Buten-Einheiten enthalten kann, durch Hydroformylierung und reduktive Aminierung mit Ammoniak, Monoaminen oder Polyaminen wie Dimethylaminopropylamin, Ethylendiamin, Diethylentriamin, Triethylentetramin oder Tetraethylenpentamin hergestellt werden können, sind insbesondere aus der Schrift EP 244 616 A2 bekannt. Geht man bei der Herstellung der Additive von Polybuten oder Polyisobuten mit überwiegend mittenständigen Doppelbindungen (meist in der β- und γ-Position) aus, bietet sich der Herstellweg durch Chlorierung und anschließende Aminierung oder durch Oxidation der Doppelbindung mit Luft oder Ozon zur Carbonyl- oder Carboxylverbindung und anschließende Aminierung unter reduktiven (hydrierenden) Bedingungen an. Zur Aminierung können hier die gleichen Amine wie oben für die reduktive Aminierung des hydroformylierten hochreaktiven Polyisobutens eingesetzt werden. Entsprechende Additive auf Basis von Polypropen sind insbesondere in der Schrift WO 94/24231 A1 beschrieben.

Weitere bevorzugte Monoaminogruppen (i) enthaltende Additive sind die Hydrierungsprodukte der Umsetzungsprodukte aus Polyisobutenen mit einem mittleren Polymerisationsgrad P = 5 bis 100 mit Stickoxiden oder Gemischen aus Stickoxiden und Sauerstoff, wie sie insbesondere in der Schrift WO 97/03946 A1 beschrieben sind.

Weitere bevorzugte Monoaminogruppen (i) enthaltende Additive sind die aus Polyisobutenepoxiden durch Umsetzung mit Aminen und nachfolgende Dehydratisierung und Reduktion der Aminoalkohole erhältlichen Verbindungen, wie sie insbesondere in der Schrift DE 196 20 262 A1 beschrieben sind.

Nitrogruppen, gegebenenfalls in Kombination mit Hydroxylgruppen, (ii) enthaltende Additive sind vorzugsweise Umsetzungsprodukte aus Polyisobutenen des mittleren Polymerisationsgrades P = 5 bis 100 oder 10 bis 100 mit Stickoxiden oder Gemischen aus Stickoxiden und Sauerstoff, wie sie insbesondere in den Schriften WO 96/03367 A1 und WO 96/03479 A1 beschrieben sind. Diese Umsetzungsprodukte stellen in der Regel Mischungen aus reinen Nitropolyisobutanen (z.B. α,β-Dinitropolyisobutan) und gemischten Hydroxynitropolyisobutanen (z.B. α-Nitro-β-hydroxypolyisobutan) dar.

Hydroxylgruppen in Kombination mit Mono- oder Polyaminogruppen (iii) enthaltende Additive sind insbesondere Umsetzungsprodukte von Polyisobutenepoxiden, erhältlich aus vorzugsweise überwiegend endständige Doppelbindungen aufweisendem Polyisobuten mit M_{N} = 300 bis 5000, mit Ammoniak, Mono- oder Polyaminen, wie sie insbesondere in der Schrift EP 476 485 A1 beschrieben sind.

Carboxylgruppen oder deren Alkalimetall- oder Erdalkalimetallsalze (iv) enthaltende Additive sind vorzugsweise Copolymere von C₂-C₄₀-Olefinen mit Maleinsäureanhydrid mit einer Gesamt-Molmasse von 500 bis 20 000, deren Carboxylgruppen ganz oder teilweise zu den Alkalimetall- oder Erdalkalimetallsalzen und ein verbleibender Rest der Carboxylgruppen mit Alkoholen oder Aminen umgesetzt sind. Solche Additive sind insbesondere aus der Schrift EP 307 815 A1 bekannt. Derartige Additive dienen hauptsächlich zur Verhinderung von Ventilsitzverschleiß und können, wie in der Schrift WO 87/01126 A1 beschrieben, mit Vorteil in Kombination mit üblichen Detergenzien, wie Poly(iso)butenaminen oder Polyetheraminen, eingesetzt werden.

Sulfonsäuregruppen oder deren Alkalimetall- oder Erdalkalimetallsalze (v) enthaltende Additive sind vorzugsweise Alkalimetall- oder Erdalkalimetallsalze eines Sulfobernsteinsäurealkylesters, wie er insbesondere in der Schrift EP 639 632 A1 beschrieben ist. Derartige Additive dienen hauptsächlich zur Verhinderung von Ventilsitzverschleiß und können mit Vorteil in Kombination mit üblichen Detergenzien, wie Poly(iso)butenaminen oder Polyetheraminen, eingesetzt werden.

Polyoxy-C₂-C₄-alkylengruppierungen (vi) enthaltende Additive sind vorzugsweise Polyether oder Polyetheramine, welche durch Umsetzung von C₂-C₆₀-Alkanolen, C₆-C₃₀-Alkandiolen, Mono- oder Di-C₂-C₃₀-alkylaminen, C₁-C₃₀-Alkylcyclohexanolen oder C₁-C₃₀-Alkylphenolen mit 1 bis 30 mol Ethylenoxid und/oder Propylenoxid und/oder Butylenoxid pro Hydroxylgruppe oder Aminogruppe und, im Falle der Polyetheramine, durch anschließende reduktive Aminierung mit Ammoniak, Monoaminen oder Polyaminen erhältlich sind. Derartige Produkte werden insbesondere in den Schriften EP 310 875 A1, EP 356 725 A1, EP 700 985 A1 und US 4,877,416 beschrieben. Im Falle von Polyethern erfüllen solche Produkte auch Trägeröleigenschaften. Typische Beispiele hierfür sind Tridecanol- oder Isotridecanotbutoxylate, Isononylphenolbutoxylate sowie Polyisobutenolbutoxylate und -propoxylate sowie die entsprechenden Umsetzungsprodukte mit Ammoniak.

Carbonsäureestergruppen (vii) enthaltende Additive sind vorzugsweise Ester aus Mono-, Di- oder Tricarbonsäuren mit langkettigen Alkanolen oder Polyolen, insbesondere solche mit einer Mindestviskosität von 2 mm2/s bei 100°C, wie sie insbesondere in der Schrift DE 38 38 918 A1 beschrieben sind. Als Mono-, Di- oder Tricarbonsäuren können aliphatische oder aromatische Säuren eingesetzt werden, als Esteralkohole beziehungsweise -polyole eignen sich vor allem langkettige Vertreter mit beispielsweise 6 bis 24 C-Atomen. Typische Vertreter der Ester sind Adipate, Phthalate, iso-Phthalate, Terephthalate und Trimellitate des iso-Octanols, iso-Nonanols, iso-Decanols und des iso-Tride. Aus Bernsteinsäureanhydrid abgeleitete Gruppierungen mit Hydroxy- und/oder Amino- und/oder Amido- und/oder Imidogruppen (viii) enthaltende Additive sind vorzugsweise entsprechende Derivate von Polyisobutenylbernsteinsäureanhydrid, welche durch Umsetzung von konventionellem oder hochreaktivem Polyisobuten mit M_{N} = 300 bis 5000 mit Maleinsäureanhydrid auf thermischen Wege oder über das chlorierte Polyisobuten erhältlich sind. Von besonderem Interesse sind hierbei Derivate mit aliphatischen Polyaminen wie Ethylendiamin, Diethylentriamin, Triethylentetramin oder Tetraethylenpentamin. Derartige Ottokraftstoffadditive sind insbesondere in der Schrift US 4,849,572 beschrieben.

Durch Mannich-Umsetzung von phenolischen Hydroxylgruppen mit Aldehyden und Mono- oder Polyaminen erzeugte Gruppierungen (ix) enthaltende Additive sind vorzugsweise Umsetzungsprodukte von polyisobutensubstituierten Phenolen mit Formaldehyd und Mono- oder Polyaminen wie Ethylendiamin, Diethylentriamin, Triethylentetramin, Tetraethylenpentamin oder Dimethylaminopropylamin. Die polyisobutenyl-substituierten Phenole können aus konventionellem oder hochreaktivem Polyisobuten mit M_{N} = 300 bis 5000 stammen. Derartige "Polyisobuten-Mannichbasen" sind insbesondere in der Schrift EP 831 141 A1 beschrieben.

Zur genaueren Definition der einzelnen aufgeführten Additive wird hier auf die Offenbarungen der oben genannten Schriften des Standes der Technik ausdrücklich Bezug genommen.

Dispergatoren als Komponente c) sind beispielsweise Imide, Amide, Ester und Ammonium- und Alkalimetallsalze von Polyisobutenbernsteinsäureanhydriden. Diese Verbindungen finden insbesondere Einsatz in Schmierölen, teilweise jedoch auch als Detergenzien in Kraftstoffzusammensetzungen.

Weitere Additive und Hilfsmittel, welche gegebenenfalls als Komponente d) der Packages vorhanden sein können, sind
organische Lösungsmittel, z.B. Alkohole, wie Methanol, Ethanol, Propanol, Isopropanol, Butanol, Isobutanol, sec-Butanol, Pentanol, Isopentanol, Neopentanol oder Hexanol, z.B. Glykole, wie 1,2-Ethylenglykol, 1,2- oder 1,3-Propylenglykol, 1,2-, 2,3- oder 1 ,4-Butylenglykol, Di- oder Triethylenglykol oder Di- oder Tripropylenglykol, z.B. Ether, wie Methyltertbutylether, 1,2-Ethylenglykolmono-oder-dimethylether, 1,2-Ethylenglykolmono- öder -diethylether, 3-Methoxypropanol, 3-Isopropoxypropanol, Tetrahydrofuran oder Dioxan, z.B. Ketone, wie Aceton, Methylethylketon oder Diacetonalkohol, z.B. Ester, wie Essigsäuremethylester, Essigsäureethylester, Essigsäurepropylester oder Essigsäurebutylester, z.B. Lactame, wie N-Methylpyrrolidinon (NMP), z.B. aliphatische oder aromatische Kohlenwasserstoffe sowie deren Gemische, wie Pentan, Hexan, Heptan, Octan, Isooctan, Petrolether, Toluol, Xylol, Ethylbenzol, Tetralin, Dekalin, Dimethylnaphthalin oder Testbenzin und z.B. Mineralöl, wie Benzin, Kerosin, Dieselöl oder Heizöl,

Korrosionsinhibitoren, beispielsweise auf Basis von zur Filmbildung neigenden Ammoniumsalzen organischer Carbonsäuren oder von heterocyclischen Aromaten bei Buntmetallkorrosionsschutz,

Antioxidantien oder Stabilisatoren, beispielsweise auf Basis von Aminen wie p-Phenylendiamin, Dicyclohexylamin oder Derivaten hiervon oder von Phenolen wie 2,4-Di-tert.-butylphenol oder 3,5-Di-tert.-butyl-4-hydroxyphenylpropionsäure,
Demulgatoren,
Antistatikmittel,
Metallocene wie Ferrocen oder Methylcyclopentadienylmangantricarbonyl,
Schmierfähigkeitsverbesserer (Lubricity-Additive) wie bestimmte Fettsäuren, Alkenylbernsteinsäureester, Bis(hydroxyalkyl)fettamine, Hydroxyacetamide oder Rizinusöl,
Amine zur Erhöhung des pH-Wertes des Kraftstoffes,
weitere, von den Verbindungen der allgemeinen Formel (I) verschiedene Markierstoffe sowie
Farbstoffe.

Die Konzentration der Komponente a), d.h. der mindestens einen Verbindung der allgemeinen Formel (I), in den erfindungsgemäßen Packages wird üblicherweise in einer solchen Höhe gewählt, dass nach Zugabe des Packages zum Mineralöl die gewünschte Konzentration an Markierungsstoff(en) darin enthalten ist. Übliche Konzentrationen der Markierstoffe im Mineralöl liegen etwa im Bereich von 0,01 bis zu einigen 10 Gew.-ppm.

Komponente b), d.h. das mindestens eine Trägeröl, ist in den Packages üblicherweise in einer Konzentration von 1 bis 50, insbesondere 5 bis 30 Gew.-%, und Komponente c), d.h. das mindestens eine Detergens und/oder der mindestens eine Dispergator, üblicherweise in einer Konzentration von 25 bis 90 Gew.-%, insbesondere 30 bis 80 Gew.-%, enthalten, jeweils bezogen auf die Gesamtmenge der Komponenten a) bis c) und gegebenenfalls d), wobei die Summe der Einzelkonzentrationen der Komponenten a) bis c) und gegebenenfalls d) sich zu 100 Gew.-% ergänzt.

Sofern als Komponente d) Korrosionsinhibitoren, Antioxidantien oder Stabilisatoren, Demulgatoren, Antistatikmittel, Metallocene, Schmierfähigkeitsverbesserer und Amine zur Absenkung des pH-Wertes des Kraftstoffes, in den Packages enthalten sind, beläuft sich deren Konzentration in der Summe üblicherweise auf nicht mehr als 10 Gew.-%, bezogen auf die Gesamtmenge des Packages (d.h. die Gesamtmenge der Komponenten a) bis c) und d)), wobei die Konzentration der Korrosionsinhibitoren und Demulgatoren üblicherweise im Bereich von jeweils etwa 0,01 bis 0,5 Gew.-% der Gesamtmenge des Packages liegt.

Sofern als Komponente d) zusätzliche (d.h. nicht bereits mit den übrigen Komponenten eingebrachte) organische Lösungsmittel in den Packages enthalten sind, beläuft sich deren Konzentration in der Summe üblicherweise auf nicht mehr als 20 Gew.-%, bezogen auf die Gesamtmenge des Packages. Diese Lösungsmittel stammen in der Regel von Lösungen der Markierstoffe und/oder Farbstoffe, welche im Hinblick auf eine genauere Dosierbarkeit -anstelle der reinen Markierstoffe und/oder Farbstoffe- den Packages zugegeben werden.

Sofern als Komponente d) weitere, von den Verbindungen der allgemeinen Formel (I) verschiedene Markierstoffe in den Packages enthalten sind, bemisst sich deren Konzentration wiederum am Gehalt, welchen diese nach Zugabe der Packages im Mineralöl aufweisen sollen. Sinngemäß gilt das zu Komponente a) Gesagte.

Sofern als Komponente d) Farbstoffe in den erfindungsgemäßen Packages enthalten sind, liegt deren Konzentration üblicherweise etwa zwischen 0,1 bis 5 Gew.-%, bezogen auf die Gesamtmenge des Packages.

Die Erfindung wird durch die Beispiele näher erläutert ohne dass die Beispiele den Gegenstand der Erfindung einschränken.

### Beispiele:

Die Herstellung der Verbindungen der allgemeinen Formel (I) in den Beispielen erfolgte nach den oben erwähnten bekannten Verfahren, die dem Fachmann aus der Literatur geläufig sind.

Tridecylamin-Isomerengemisch: Verkaufsprodukt der BASF Aktiengesellschaft.

### Beispiel 1: Herstellung von 1,4-Diamino-N-tridecyl-2,3-anthrachinondicarboximid (Isomerengemisch)

12,8 g (0,042 mol) 1,4-Diamino-2,3-anthrachinondicarboximid wurden in 64 g o-Dichlorbenzol suspendiert. Die Reaktionsmischung wurde auf 140°C erhitzt, kurz (ca. 10 min) gerührt und anschließend auf 60 °C abgekühlt. Bei 60 °C wurden 19,9 g (0,1 mol) Tridecylamin-Isomerengemisch zur Reaktionsmischung dazugegeben. Die Reaktionsmischung wurde nun auf 120°C erhitzt und ca. 7 h bei dieser Temperatur gehalten. Danach wurde die Mischung erneut auf 60 °C abgekühlt und mit 2,0 g (0,01 mol) Tridecylamin-Isoinerengemisch versetzt. Anschließend wurde das Reaktionsgemisch wieder auf 120°C erwärmt und ca. 3,5 h bei dieser Temperatur gehalten. Danach erfolgte eine erneute Zugabe von 2,0 g (0,01 mol) Tridecylamin-Isomerengemisch. Nach weiteren 17,5 h bei 120°C wurde die Reaktionsmischung auf Raumtemperatur abgekühlt und filtriert, wobei ein Feststoff zurückblieb, der gewaschen und im Vakuumtrockenschrank getrocknet wurde. Aus dem Filtrat wurde durch Zugabe von ca. 200 ml Methanol weiterer Feststoff ausgefällt, der abfiltriert, gewaschen und im Vakuumtrockenschrank getrocknet wurde.
Die Reinigung der vereinigten Feststoffe erfolgte durch Säulenchromatographie an Kieselgel mit Dichlormethan als Laufmittel. Nach Entfernen des Lösungsmittels wurden 11,3 g blauer Feststoff erhalten.
Als spektroskopischen Daten (Absorptionspektrum) des Produktes wurden bestimmt: UV/Vis: λₘₐₓ (ε) = 676 nm (15400) in Toluol

### Beispiel 2: Detektion eines Markierstoffes durch Nachweis der Fluoreszenz in THF

Der Markierstoff 1,4-Diamino-N-isopropyl-2,3-anthrachinondicarboximid 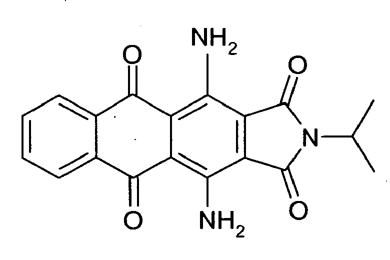 wurde in verschiedenen Konzentrationen in Tetrahydrofuran (THF) gelöst und durch Messung der angeregten Fluoreszenz nachgewiesen. Zu diesem Zweck wurde die Lösung des Markierstoffs mit Hilfe einer Laserdiode, welche eine Leistung von 3 mW und eine Anregungswellenlänge von 660 nm besitzt, angeregt. Die Fluoreszenz wurde senkrecht zum Anregungungsstrahl durch ein kommerzielles Kantenfilter mit einer Kantenwellenlänge von 695 nm (Langpaß) mittels Si-Photodiode integral detektiert. Es wurden folgende Resultate erhalten:

| Konzentration [ppb] | SKT |
|---|---|
| 1000 | 2,110 |
| 500 | 1,150 |
| 200 | 0,412 |
| 100 | 0,194 |
| 50 | 0,100 |
| 20 | 0,050 |
| 10 | 0,021 |
| 0 | 0,001 |

Die gemessenen relativen Fluoreszenzintensitäten (Skalenteile, SKT) korrelieren linear sehr gut mit den eingesetzten Konzentrationen. Das Quadrat des Korrelationskoeffizienten beträgt: 0,998.

### Beispiel 3: Detektion eines Markierstoffes durch Nachweis der Fluoreszenz in Benzin

Der Markierstoff aus Beispiel 1 wurde in Benzin der Marke ARAL® Ultimate gelöst. Es wurden folgende Ergebnisse für die Fluoreszenzintensitäten erhalten:

| Konzentration [ppb] | SKT |
|---|---|
| 1000 | 1,510 |
| 500 | 0,657 |
| 200 | 0,271 |
| 100 | 0,141 |
| 50 | 0,098 |
| 20 | 0,036 |
| 10 | 0,015 |
| 0 | 0,000 |

Auch in diesem Beispiel korreliert das Fluoreszenzsignal (relative Intensität: Skalenteile, SKT) linear gut mit der Konzentration. Das Quadrat des Korrelationskoeffizienten beträgt: 0.995.

### Beispiel 4: Absorptionswellenlängen einiger Markierstoffe

Die Markierstoffe wurden in Methylenchlorid (MCL) gelöst und das langwelligste Absorptionsmaximum λₘₐₓ der einzelnen Stoffe bestimmt.

| Markierstoff | λₘₐₓ (MCL) [nm] |
|---|---|
| 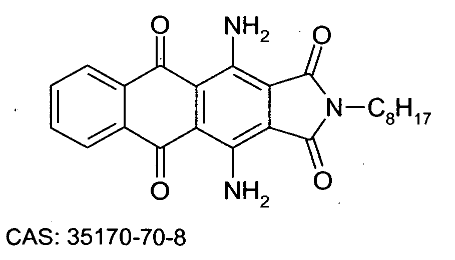 | 668 |
| 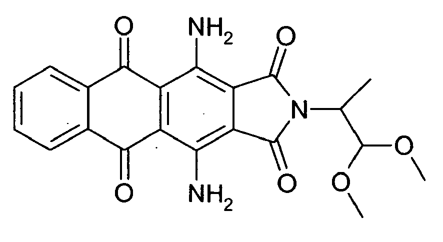 | 675 |
| 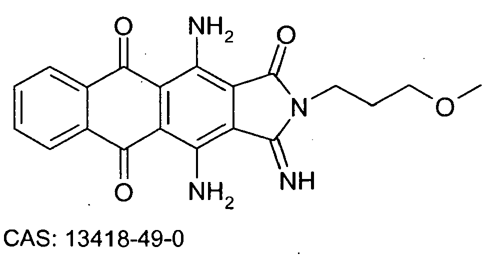 | 659 |
| 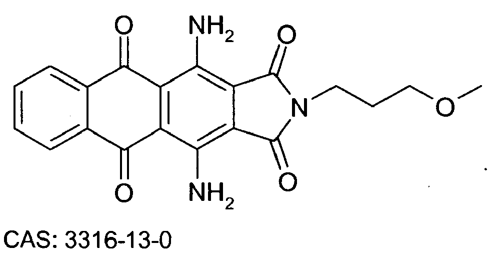 | 668 |

## Patentansprüche

1. Verwendung von Verbindungen der allgemeinen Formel (I) 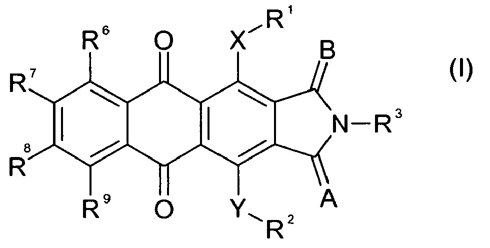 als Markierstoffe für Flüssigkeiten, wobei die Symbole folgende Bedeutung haben
X, Y unabhängig voneinander, gleich oder verschieden, O, NR⁴, Heterocyclen,
A, B unabhängig voneinander, gleich oder verschieden, O, NR⁵,
M Alkalimetalle,
R¹, R² unabhängig voneinander, gleich oder verschieden, H, C₁-C₂₀-Alkyl, C₂-C₂₀-Alkenyl, C₂-C₂₀-Alkinyl, C₃-C₁₅-Cycloalkyl, Aryl, Heterocyclen,
R³ C₁-C₂₀-Alkyl, C₁-C₂₀-Alkylcarbonyl, C₂-C₂₀-Alkenyl, C₂-C₂₀-Alkenylcarbonyl , C₂-C₂₀-Alkinyl, C₂-C₂₀-Alkinylcarbonyl, C₃-C₁₅-Cycloalkyl, C₃-C₁₅-Cycloalkylcarbonyl, Aryl, Arylcarbonyl, Heterocyclen,
R⁴ H, C₁-C₂₀-Alkyl, C₂-C₂₀-Alkenyl, C₂-C₂₀-Alkinyl, C₃-C₁₅-Cycloalkyl , Aryl, Heterocyclen,
R⁵ H, C₁-C₂₀-Alkyl, C₂-C₂₀-Alkenyl, C₂-C₂₀-Alkinyl,
R⁶, R⁷, R⁸, R⁹ C₃-C₁₅-Cycloalkyl, Aryl, Heterocyclen, unabhängig voneinander, gleich oder verschieden, H, C₁-C₂₀-Alkyl, C₁-C₂₀-Alkylcarbonyl, C₁-C₂₀-Alkoxy, C₁-C₂₀- Alkoxycarbonyl, C₂-C₂₀-Alkenyl, C₂-C₂₀-Alkenylcarbonyl , C₂- C₂₀-Alkinyl, C₂-C₂₀-Alkinylcarbonyl, C₃-C₁₅-Cycloalkyl, C₃-C₁₅-Cycloalkylcarbonyl, Aryl, Arylcarbonyl, Aryloxy, Aryloxycarbonyl, Heterocyclen, NR¹R², Halogen, CN, NO₂,
wobei die Substituenten R¹, R², R³, R⁴, R⁵, R⁶, R⁷, R⁸ oder R⁹ jeweils an beliebiger Position durch ein oder mehrere Heteroatome unterbrochen sein können, wobei die Anzahl dieser Heteroatome nicht mehr als 10, bevorzugt nicht mehr als 8, ganz besonders bevorzugt nicht mehr als 5 und insbesondere nicht mehr als 3 beträgt, und/oder jeweils an beliebiger Position, allerdings nicht mehr als fünfmal, bevorzugt nicht mehr als viermal und besonders bevorzugt nicht mehr als dreimal, durch NR¹R², CONR¹R², COOM, COOR¹, SO₃M, SO₃R¹, CN, NO₂, C₁-C₂₀-Alkyl, C₁-C₂₀-Alkoxy, Aryl, Aryloxy, Heterocyclen, Heteroatome oder Halogen substituiert sein können, wobei diese ebenfalls maximal zweimal, bevorzugt maximal einmal mit den genannten Gruppen substituiert sein können.

2. Verwendung gemäß Anspruch 1, wobei die Symbole folgende Bedeutung haben,
X, Y unabhängig voneinander, gleich oder verschieden, O, NR⁴,
A, B unabhängig voneinander, gleich oder verschieden, NH, O,
R¹, R² gleich oder verschieden H, C₁-C₂₀-Alkyl, Aryl,
R³ C₁-C₂₀-Alkyl, C₃-C₁₅-Cycloalkyl, Aryl, Heterocyclen,
R⁴, H, C₁-C₂₀-Alkyl, Aryl, Heterocyclen,
R6, R⁷, R⁸, R⁹ unabhängig voneinander, gleich oder verschieden, H, C₁-C₂₀-Alkyl, C₁-C₂₀-Alkoxy, C₃-C₁₅-Cycloalkyl, Aryl, Aryloxy, NR¹R², Halogen, CN, NO₂.

3. Verwendung gemäß Anspruch 1, wobei die Symbole folgende Bedeutung haben,
X, Y beide gleich NR⁴,
A, B unabhängig voneinander, gleich oder verschieden, NH, O,
R¹, R² H,
R³ C₁-C₂₀-Alkyl, C₃-C₁₅-Cycloalkyl, Aryl,
R⁴, H,
R⁶, R⁷, R⁸, R⁹ unabhängig voneinander, gleich oder verschieden, H, C₁-C₂₀-Alkyl, C₁-C₂₀-Alkoxy, Aryl, Aryloxy, NR¹R², F, Cl, Br, CN, NO₂.

4. Verwendung gemäß den Ansprüchen 1 bis 3, wobei die Flüssigkeit ein Öl ist.

5. Verwendung gemäß den Ansprüchen 1 bis 3, wobei die Flüssigkeit ein Mineralöl ist.

6. Verwendung gemäß den Ansprüchen 1 bis 3, wobei die Flüssigkeit ein Additiv-Konzentrat ist.

7. Flüssigkeit, enthaltend mindestens eine Verbindung der allgemeinen Formel (I), gemäß den Ansprüchen 1 bis 3 als Markierstoff.

8. Flüssigkeit gemäß Anspruch 7, wobei die Flüssigkeit ein Öl ist.

9. Flüssigkeit gemäß Anspruch 7, wobei die Flüssigkeit ein Mineralöl ist.

10. Flüssigkeit gemäß Anspruch 7, wobei die Flüssigkeit ein Additiv-Konzentrat ist.

11. Verfahren zur Detektion von Markierstoffen in Flüssigkeiten, welche mindestens eine Verbindung der allgemeinen Formel (I) gemäß den Ansprüchen 1 bis 3 in einer Menge enthalten, die ausreichend ist, bei Bestrahlung mit Strahlung einer geeigneten Wellenlänge eine nachweisbare Fluoreszenz anzuregen, wobei
a) die Flüssigkeit mit elektromagnetischer Strahlung einer Wellenlänge von 500 bis 900 nm bestrahlt wird und
b) die angeregte Fluoreszenzstrahlung mit einer Vorrichtung zum Nachweis von Strahlung im Bereich von 500 bis 1000 nm detektiert wird.

12. Verfahren zur Detektion von Markierstoffen in Flüssigkeiten, welche mindestens eine Verbindung der allgemeinen Formel (I) gemäß den Ansprüchen 1 bis 3 in einer Menge enthalten, die ausreichend ist, bei Bestrahlung mit Strahlung einer geeigneten Wellenlänge eine nachweisbare Absorption zu zeigen, wobei
a) die Flüssigkeit mit elektromagnetischer Strahlung einer Wellenlänge von 500 bis 900 nm bestrahlt wird und
b) die Absorption der Strahlung a) mit einer Vorrichtung zum Nachweis von Strahlung im Bereich von 500 bis 900 nm detektiert wird.

13. Verfahren gemäß den Ansprüchen 11 oder 12, wobei die Flüssigkeit ein Öl ist.

14. Verfahren gemäß den Ansprüchen 11 oder 12, wobei die Flüssigkeit ein Mineralöl ist.

15. Verfahren gemäß den Ansprüchen 11 oder 12, wobei die Flüssigkeit ein Additiv-Konzentrat ist.

16. Verfahren zur Identifizierung von Flüssigkeiten, welche mindestens eine Verbindung der allgemeinen Formel (I) gemäß den Ansprüchen 1 bis 3 in einer Menge enthalten, die ausreichend ist, bei Bestrahlung mit Strahlung einer geeigneten Wellenlänge eine nachweisbare Fluoreszenz anzuregen, wobei
a) die Flüssigkeit mit elektromagnetischer Strahlung einer Wellenlänge von 500 bis 900 nm bestrahlt wird und
b) die Absorption der elektromagnetischen Strahlung a) mit einer Vorrichtung zum Nachweis von Strahlung detektiert wird und
c) die angeregte Fluoreszenzstrahlung mit einer Vorrichtung zum Nachweis von Strahlung im Bereich von 500 bis 1000 nm detektiert wird und
d) die Flüssigkeit mit Hilfe der Absorption b) und/oder Fluoreszenz c) identifi ziert wird und
e) die Konzentration der Verbindung der allgemeinen Formel (I) in der Flüssigkeit mit Hilfe der Fluoreszenzstrahlung c) bestimmt wird.

17. Verfahren gemäß Anspruch 16, wobei die Flüssigkeit ein Öl ist.

18. Verfahren gemäß Anspruch 16, wobei die Flüssigkeit ein Mineralöl ist.

19. Verfahren gemäß Anspruch 16, wobei die Flüssigkeit ein Additiv-Konzentrat ist.

20. Verbindungen der allgemeinen Formel (I), wobei die Verbindungen 1,4-Diamino-N-tridecyl-2,3-anthrachinondicarboximide sind und der Tridecyl-Substituent auch ein Isomerengemisch verschiedener Tridecyle sein kann.

21. Verbindung der allgemeinen Formel (I), wobei die Verbindung 1,4-Diamino-N-(2,6-diisopropylphenyl)-2,3-anthrachinondicarboximid ist.

22. Verbindungen der allgemeinen Formel (I), wobei die Verbindungen 1,4-Diamino-N-(4-dodecylphenyl)-2,3-anthrachinondicarboximide sind und der Dodecyl-Substituent auch ein Isomerengemisch verschiedener Dodecyle sein kann.

23. Verbindung der Formel (la)

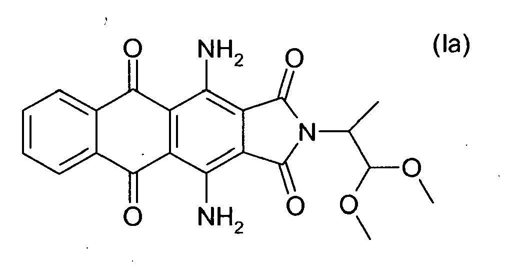

## Claims

1. The use of compounds of the general formula (I) 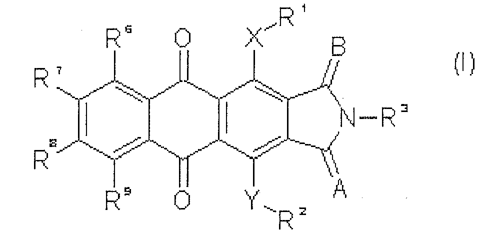 as markers for liquids, where the symbols are each defined as follows:
X, Y are each independently, identically or differently, O, NR⁴, heterocycles,
A, B are each independently, identically or differently, O, NR⁵,
M are alkali metals,
R¹, R² are each independently, identically or differently, H, C₁-C₂₀-alkyl, C₂-C₂₀-alkenyl, C₂-C₂₀-alkynyl, C₃-C₁₅- cycloalkyl, aryl, heterocycles,
R³ is C₁-C₂₀-alkyl, C₁-C₂₀-alkylcarbonyl, C₂-C₂₀-alkenyl, C₂-C₂₀-alkenylcarbonyl, C₂-C₂₀-alkynyl, C₂-C₂₀- alkynylcarbonyl, C₃-C₁₅-cycloalkyl, C₃-C₁₅- cycloalkylcarbonyl, aryl, arylcarbonyl, heterocycles,
R⁴ is H, C₁-C₂₀-alkyl, C₂-C₂₀-alkenyl, C₂-C₂₀-alkynyl, C₃-C₁₅-cycloalkyl, aryl, heterocycles,
R⁵ is H, C₁-C₂₀-alkyl, C₂-C₂₀-alkenyl, C₂-C₂₀-alkynyl, C₃-C₁₅-cycloalkyl, aryl, heterocycles,
R⁶, R⁷, R⁸, R⁹ are each independently, identically or differently, H, C₁-C₂₀-alkyl, C₁-C₂₀-alkylcarbonyl, C₁-C₂₀-alkoxy, C₁-C₂₀- alkoxycarbonyl, C₂-C₂₀-alkenyl, C₂-C₂₀-alkenylcarbonyl , C₂-C₂₀-alkynyl, C₂-C₂₀-alkynylcarbonyl, C₃-C₁₅-cycloalkyl, C₃-C₁₅-cycloalkylcarbonyl, aryl, arylcarbonyl, aryloxy, aryloxycarbonyl, heterocycles, NR¹R², halogen, CN, NO₂,
where the substituents R¹, R², R³, R⁴, R⁵, R⁶, R⁷, R⁸ or R⁹ may each be interrupted at any position by one or more heteroatoms, where the number of these heteroatoms is not more than 10, preferably not more than 8, more preferably not more than 5 and especially not more than 3, and/or may be substituted in each case at any position, but not more than five times, preferably not more than four times and more preferably not more than three times, by NR¹R², CONR¹R², COOM, COOR¹, SO₃M, SO₃R¹, CN, NO₂, C₁-C₂₀-alkyl, C₁-C₂₀-alkoxy, aryl, aryloxy, heterocycles, heteroatoms or halogen, where these may likewise be substituted not more than twice, preferably not more than once, by the groups mentioned.

2. The use according to claim 1, wherein the symbols are each defined as follows:
X, Y are each independently, identically or differently, O, NR⁴,
A, B are each independently, identically or differently, NH, O,
R¹, R² are identically or differently H, C₁-C₂₀-alkyl, aryl,
R³ is C₁-C₂₀-alkyl, C₃-C₁₅-cycloalkyl, aryl, heterocycles,
R⁴ is H, C₁-C₂₀-alkyl, aryl, heterocycles,
R⁶, R⁷, R⁸, R⁹ are each independently, identically or differently, H, C₁-C₂₀-alkyl, C₁-C₂₀-alkoxy, C₃-C₁₅-cycloalkyl, aryl, aryloxy, NR¹R², halogen, CN, NO₂.

3. The use according to claim 1, wherein the symbols are each defined as follows:
X, Y are both identically NR⁴,
A, B are each independently, identically or differently, NH, O,
R¹, R² are each H,
R³ is C₁-C₂₀-alkyl, C₃-C₁₅-cycloalkyl, aryl,
R⁴ is H,
R⁶, R⁷, R⁸, R⁹ are each independently, identically or differently, H, C₁-C₂₀-alkyl, C₁-C₂₀-alkoxy, aryl, aryloxy, NR¹R², F, Cl, Br, CN, NO₂.

4. The use according to claims 1 to 3, wherein the liquid is an oil.

5. The use according to claims 1 to 3, wherein the liquid is a mineral oil.

6. The use according to claims 1 to 3, wherein the liquid is an additive concentrate.

7. A liquid comprising at least one compound of the general formula (I) according to claims 1 to 3 as a marker.

8. The liquid according to claim 7, wherein the liquid is an oil.

9. The liquid according to claim 7, wherein the liquid is a mineral oil.

10. The liquid according to claim 7, wherein the liquid is an additive concentrate.

11. A method for detecting markers in liquids which comprise at least one compound of the general formula (I) according to claims 1 to 3 in an amount which is sufficient to excite detectable fluorescence on irradiation with radiation of a suitable wavelength, wherein
a) the liquid is irradiated with electromagnetic radiation of a wavelength of from 500 to 900 nm and
b) the excited fluorescence radiation is detected with a device for detecting radiation in the range from 500 to 1000 nm.

12. A method for detecting markers in liquids which comprise at least one compound of the general formula (I) according to claims 1 to 3 in an amount which is sufficient to exhibit detectable absorption on irradiation with radiation of a suitable wavelength, wherein
a) the liquid is irradiated with electromagnetic radiation of a wavelength of from 500 to 900 nm and
b) the absorption of the radiation a) is detected with a device for detecting radiation in the range from 500 to 900 nm.

13. The method according to claims 11 and 12, wherein the liquid is an oil.

14. The method according to claims 11 and 12, wherein the liquid is a mineral oil.

15. The method according to claims 11 and 12, wherein the liquid is an additive concentrate.

16. A method for identifying liquids which comprise at least one compound of the general formula (I) according to claims 1 to 3 in an amount which is sufficient to excite detectable fluorescence on irradiation with radiation of a suitable wavelength, wherein
a) the liquid is irradiated with electromagnetic radiation of a wavelength of from 500 to 900 nm and
b) the absorption of the electromagnetic radiation a) is detected with a device for detecting radiation and
c) the excited fluorescence radiation is detected with a device for detecting radiation in the range from 500 to 1000 nm and
d) the liquid is identified with the aid of the absorption b) and/or fluorescence c) and
e) the concentration of the compound of the general formula (I) in the liquid is determined with the aid of fluorescence radiation c).

17. The method according to claim 16, wherein the liquid is an oil.

18. The method according to claim 16, wherein the liquid is a mineral oil.

19. The method according to claim 16, wherein the liquid is an additive concentrate.

20. A compound of the general formula (I), which is a 1,4-diamino-N-tridecyl-2,3-anthraquinonedicarboximide and the tridecyl substituent may also be an isomer mixture of different tridecyls.

21. A compound of the general formula (I), which is a 1,4-diamino-N-(2,6-diisopropylphenyl)-2,3-anthraquinonedicarboximide.

22. A compound of the general formula (I), which is a 1,4-diamino-N-(4-dodecylphenyl)-2,3-anthraquinonedicarboximide and the dodecyl substituent may also be an isomer mixture of different dodecyls.

23. A compound of the formula (Ia)

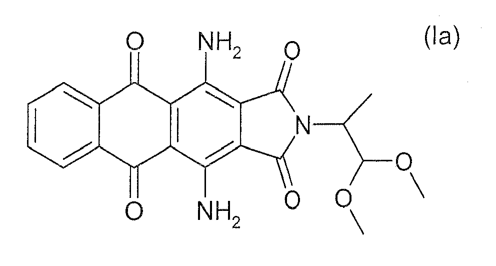

## Revendications

1. Utilisation de composés de formule générale (I) 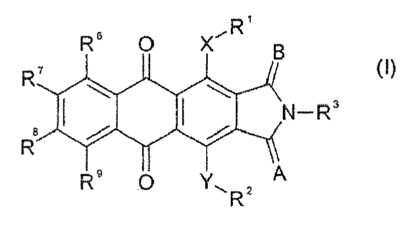 en tant que marqueurs pour liquides, les symboles ayant les significations suivantes
X, Y sont identiques ou différents et représentent, indépendamment l'un de l'autre, O, NR⁴, des hétérocycles,
A, B sont identiques ou différents et représentent, indépendamment l'un de l'autre, O, NR⁵,
M représente des métaux alcalins,
R¹, R² sont identiques ou différents et représentent, indépendamment l'un de l'autre, H, un groupe alkyle en C₁-C₂₀, alcényle en C₂-C₂₀, alcynyle en C₂-C₂₀, cycloalkyle en C₃-C₁₅, aryle, des hétérocycles,
R³ représente un groupe alkyle en C₁-C₂₀, alkyl(C₁-C₂₀)carbonyle, alcényle en C₂-C₂₀, alcényl(C₂-C₂₀)carbonyle, alcynyle en C₂-C₂₀, alcynyl(C₂-C₂₀)carbonyle, cycloalkyle en C₃-C₁₅, cycloalkyl(C₃-C₁₅)carbonyle, aryle, arylcarbonyle, des hétérocycles,
R⁴ représente H, un groupe alkyle en C₁-C₂₀, alcényle en C₂-C₂₀, alcynyle en C₂-C₂₀, cycloalkyle en C₃-C₁₅, aryle, des hétérocycles,
R⁵ représente H, un groupe alkyle en C₁-C₂₀, alcényle en C₂-C₂₀, alcynyle en C₂-C₂₀, cycloalkyle en C₃-C₁₅, aryle, des hétérocycles,
R⁶, R⁷, R⁸, R⁹ sont identiques ou différents et représentent, indépendamment les uns des autres, H, un groupe alkyle en C₁-C₂₀, alkyl(C₁-C₂₀)carbonyle, alcoxy en C₁-C₂₀, alcoxy(C₁-C₂₀)carbonyle, alcényle en C₂-C₂₀, alcényl(C₂-C₂₀)carbonyle, alcynyle en C₂-C₂₀, alcynyl-(C₂-C₂₀) carbonyle, cycloalkyle en C₃-C₁₅, cycloalkyl(C₃-C₁₅)carbonyle, aryle, arylcarbonyle, aryloxy, aryloxycarbonyle, des hétérocycles, NR¹R², un atome d'halogène, CN, NO₂,
les substituants R¹, R², R³, R⁴, R⁵, R⁶, R⁷, R⁸ ou R⁹ pouvant être interrompus chacun en position quelconque par un ou plusieurs hétéroatomes, le nombre de ces hétéroatomes n'étant pas supérieur à 10, de préférence pas supérieur à 8, de façon particulièrement préférée, pas supérieur à 5 et en particulier pas supérieur à 3, et/ou pouvant être substitués chacun en position quelconque, mais pas plus de cinq fois, de préférence pas plus de quatre fois et de façon particulièrement préférée pas plus de trois fois, par NR¹R², CONR¹R², COOM, COOR¹, SO₃M, SO₃R¹, CN, NO₂, des groupes alkyle en C₁-C₂₀, alcoxy en C₁-C₂₀, aryle, aryloxy, des hétérocycles, des hétéroatomes ou des atomes d'halogène, ces groupes pouvant également être substitués au maximum deux fois, de préférence au maximum une fois par les groupes nommés.

2. Utilisation selon la revendication 1, dans laquelle les symboles ont les significations suivantes X, Y sont identiques ou différents et représentent, indépendamment l'un de l'autre, O, NR⁴
A, B sont identiques ou différents et représentent, indépendamment l'un de l'autre, NH, O,
R¹, R² sont identiques ou différents et représentent H, un groupe alkyle en C₁-C₂₀, aryle,
R³ représente un groupe alkyle en C₁-C₂₀, cycloalkyle en C₃-C₁₅, aryle, des hétérocycles,
R⁴ représente H, un groupe alkyle en C₁-C₂₀, aryle, des hétérocycles,
R⁶, R7, R⁸, R⁹ sont identiques ou différents et représentent, indépendamment les uns des autres, H, un groupe alkyle en C₁-C₂₀, alcoxy en C₁-C₂₀, cycloalkyle en C₃-C₁₅, aryle, aryloxy, NR¹R², un atome d'halogène, CN, NO₂.

3. Utilisation selon la revendication 1, dans laquelle les symboles ont les significations suivantes X, Y représentent l'un et l'autre NR⁴,
A, B sont identiques ou différents et représentent, indépendamment l'un de l'autre, NH, O,
R¹, R² représentent H,
R³ représente un groupe alkyle en C₁-C₂₀, cycloalkyle en C₃-C₁₅, aryle,
R⁴ représente H,
R⁶, R⁷, R⁸, R⁹ sont identiques ou différents et représentent, indépendamment les uns des autres, H, un groupe alkyle en C₁-C₂₀, alcoxy en C₁-C₂₀, aryle, aryloxy, NR¹R², F, Cl, Br, CN, NO₂.

4. Utilisation selon les revendications 1 à 3, dans laquelle le liquide est une huile.

5. Utilisation selon les revendications 1 à 3, dans laquelle le liquide est une huile minérale.

6. Utilisation selon les revendications 1 à 3, dans laquelle le liquide est un concentré d'additif.

7. Liquide, contenant au moins un composé de formule générale (I) selon les revendications 1 à 3, en tant que marqueur.

8. Liquide selon la revendication 7, le liquide étant une huile.

9. Liquide selon la revendication 7, le liquide étant une huile minérale.

10. Liquide selon la revendication 7, le liquide étant un concentré d'additif.

11. Procédé pour la détection de marqueurs dans des liquides, qui contiennent au moins un composé de formule générale (I) selon les revendications 1 à 3, en une quantité qui est suffisante pour exciter une fluorescence détectable lors d'irradiation avec un rayonnement d'une longueur d'onde appropriée,
a) le liquide étant irradié avec un rayonnement électromagnétique d'une longueur d'onde de 500 à 900 nm et
b) le rayonnement de fluorescence excité étant détecté à l'aide d'un dispositif pour la détection d'un rayonnement dans la plage de 500 à 1 000 nm.

12. Procédé pour la détection de marqueurs dans des liquides, qui contiennent au moins un composé de formule générale (I) selon les revendications 1 à 3, en une quantité qui est suffisante pour manifester une absorption détectable lors d'irradiation avec un rayonnement d'une longueur d'onde appropriée,
a) le liquide étant irradié avec un rayonnement électromagnétique d'une longueur d'onde de 500 à 900 nm et
b) l'absorption du rayonnement a) étant détectée à l'aide d'un dispositif pour la détection d'un rayonnement dans la plage de 500 à 900 nm.

13. Procédé selon la revendication 11 ou 12, dans lequel le liquide est une huile.

14. Procédé selon la revendication 11 ou 12, dans lequel le liquide est une huile minérale.

15. Procédé selon la revendication 11 ou 12, dans lequel le liquide est un concentré d'additif.

16. Procédé pour l'identification de liquides qui contiennent au moins un composé de formule générale (I) selon les revendications 1 à 3, en une quantité qui est suffisante pour exciter une fluorescence détectable lors d'irradiation avec un rayonnement d'une longueur d'onde appropriée,
a) le liquide étant irradié avec un rayonnement électromagnétique d'une longueur d'onde de 500 à 900 nm et
b) l'absorption du rayonnement électromagnétique a) étant détectée à l'aide d'un dispositif pour la détection d'un rayonnement et
c) le rayonnement de fluorescence excité étant détecté à l'aide d'un dispositif pour la détection d'un rayonnement dans la plage de 500 à 1 000 nm et
d) le liquide étant identifié à l'aide de l'absorption b) et/ou de la fluorescence c) et
e) la concentration du composé de formule générale (I) dans le liquide étant déterminée à l'aide du rayonnement de fluorescence c).

17. Procédé selon la revendication 16, dans lequel le liquide est une huile.

18. Procédé selon la revendication 16, dans lequel le liquide est une huile minérale.

19. Procédé selon la revendication 16, dans lequel le liquide est un concentré d'additif.

20. Composés de formule générale (I), les composés étant des 1,4-diamino-N-tridécyl-2,3-anthraquinone-dicarboximides et le substituant tridécyle pouvant également être un mélange d'isomères de divers tridécyles.

21. Composé de formule générale (I), le composé étant le 1,4-diamino-N-(2,6-diisopropylphényl)-2,3-anthraquinonedicarboximide.

22. Composés de formule générale (I), les composés étant des 1,4-diamino-N-(4-dodécylphényl)-2,3-anthraquinonedicarboximides et le substituant dodécyle pouvant également être un mélange d'isomères de divers dodécyles.

23. Composé de formule (Ia)
